(19) Europäisches Patentamt
European Patent Office
Office européen des brevets

(11) **EP 0 862 417 B1**

(12) **EUROPEAN PATENT SPECIFICATION**

(45) Date of publication and mention
of the grant of the patent:
**26.02.2003 Bulletin 2003/09**

(51) Int Cl.⁷: **A61K 9/08**, A61K 31/19,
A61K 31/245, A61K 31/52,
A61K 31/54, A61K 47/32,
A61K 47/38, A61K 47/00,
A61K 9/06

(21) Application number: **96935056.0**

(22) Date of filing: **21.10.1996**

(86) International application number:
**PCT/GB96/02582**

(87) International publication number:
**WO 97/017062 (15.05.1997 Gazette 1997/21)**

(54) **PHARMACEUTICAL COMPOSITION FOR TOPICAL APPLICATION**

ARZNEIMITTEL ZUR TOPISCHEN ANWENDUNG

COMPOSITION PHARMACEUTIQUE POUR APPLICATION TOPIQUE

(84) Designated Contracting States:
**AT CH DE ES FR GB GR IT LI**

(30) Priority: **08.11.1995 GB 9522885**

(43) Date of publication of application:
**09.09.1998 Bulletin 1998/37**

(73) Proprietor: **Reckitt Benckiser Healthcare (UK) Limited**
**Slough, Berkshire SL1 3UH (GB)**

(72) Inventors:
• **POILE, Steven**
**East Yorkshire DN14 7EW (GB)**
• **HARRISON, Julian, Earle**
**East Yorkshire HU17 8RX (GB)**

(74) Representative: **Brown, Andrew Stephen et al**
**Reckitt Benckiser plc,**
**Group Patents Department,**
**Dansom Lane**
**Hull HU8 7DS (GB)**

(56) References cited:
**EP-A- 0 151 953**        **EP-A- 0 271 332**
**EP-A- 0 271 983**        **WO-A-94/15614**
**US-A- 4 897 269**

• DATABASE WPI Section Ch, Week 8649 Derwent Publications Ltd., London, GB; Class B05, AN 86-322218 XP002027301 & JP 61 238 724 A (HOKURIKU PHARM KK) , 24 October 1986
• DATABASE WPI Section Ch, Week 9651 Derwent Publications Ltd., London, GB; Class A14, AN 96-514906 XP002027302 & JP 08 268 892 A (YUWA SHOJI YG) , 15 October 1996

**Description**

The present invention relates to a pharmaceutical composition and in particular to a composition for topical application to the human or animal body.

It has long been known that in order to effectively deliver therapeutic levels of active material topically, either for local or systemic effect, there is a need to optimise the delivery system to maximise percutaneous penetration.

A number of solutions have been proposed with varying degrees of success. These include use of penetration enhancers, iontophoresis, phonophoresis and supersaturation. Whilst supersaturated solutions have been clearly demonstrated as being effective in promoting percutaneous penetration they are difficult to use because they are not very stable. Thus, solutions of the active agents may only be made supersaturated a short time before application to the skin, which is difficult if they are to be used by general consumers.

One solution has been to create a supersaturated solution of the active agent from a subsaturated solution of the drug in a mixture of a volatile and a non-volatile solvents. The mechanism behind this approach is that, when applied topically, the volatile solvent rapidly evaporates causing the drug concentration remaining in the non-volatile solvent to increase to a supersaturated level. This increase in drug concentration to supersaturation has been found to increase the rate of drug penetration into the skin.

However, a disadvantage of this system is that the volatile solvents (e.g. ethanol) may cause damage to the skin lipid membrane and may also be taken up by the body. Also, packaging has to be sophisticated enough to prevent evaporation of the volatile solvents, which leads to long term storage difficulties.

Another method to produce supersaturated compositions for the percutaneous penetration of active agents has been to produce a composition for topical application made up of two liquid phases, one containing the drug which has been dissolved in that phase and the other, which may be physically and/or chemically different from the first (but miscible with it), optionally also containing the same drug dissolved therein. The concentration of drug in each phase is such that, on admixture of the phases, the resultant total drug concentration is greater than the saturated drug solubility in the initially formed mixture of phases, thereby producing a mixture supersaturated with the drug. This enables improved drug penetration to be obtained by creating a supersaturated drug solution using a two phase composition mixed *in situ* without the need for the evaporation of volatile solvents.

However, all two phase compositions suffer from the disadvantage of requiring sophisticated packaging technology to ensure that the two phases are kept apart prior to application. Furthermore there is a need for accurate dose administration of each phase upon application, concomitant with a thorough mixing during application. This approach therefore has limited application due to the cost and sophistication of the necessary packaging technology.

We have now found that supersaturated compositions may be produced *in situ* on the skin without the drawbacks associated with the above described volatile solvent or two-phase compositions. It is known that many pharmaceutically active substances have very different solubilities in particular liquids depending upon the pH conditions. Thus if such an active is dissolved in a particular solvent at one pH, and the pH is changed, the resultant decrease in solubility may be sufficient to produce a supersaturated solution.

It has long been known that skin has an acid pH, often referred to as the "acid mantle" (the accepted pH being around 5.5 but varying from 4.5 to 6.5) which has been measured and documented extensively (Noble W.C. 1981, Microbiology of Human Skin, Lloyd-Luke Ltd). Whilst much work has been conducted to establish the pH of the acid mantle, many workers agree that it is well established that the skin has a significant buffering capacity, maintained by fatty acids, proteins and the lactic acid/lactate system present in the skin. In addition, this skin acidity is maintained in spite of the neutral or slightly alkaline pH of the dermis. We have found that solutions having a pH of 7.5 or higher will be reduced to a pH of 7 or lower (more often 6.5) after 2 hours or less contact with the skin; and that solutions having a pH of 3.5 or lower will be increased to a pH or 4 (often 4.5 or greater) after two hours or less contact with the skin.

We have now further found that by bringing acidic or alkaline solutions of appropriate concentrations of active agents into contact with the skin, the skin's innate ability to buffer such applied liquids will be sufficient to cause a solubility change, and therefore produce a supersaturated solution.

There is therefore provided a pharmaceutical composition for topical application comprising

    i) a pharmaceutically active agent, and

    ii) a pharmaceutically acceptable vehicle,

the pharmaceutical composition having a pH of 7 to 12 or a pH of 2 to 4, characterised in that the pharmaceutically active agent is dissolved at or below its saturation concentration and that the composition becomes supersaturated when the pH is changed to below 7 or greater than 4.

Such compositions may be used to produce supersaturated solutions on the skin without the need for the addition of a second phase composition; and without the use of volatile solvents.

The topical pharmaceutical compositions of the invention are single phase compositions.

The topical pharmaceutical compositions of the invention are suitable to provide active agents to act locally (i.e. at the site of application) or systemically (e.g. transdermal application).

There is further provided a process for the preparation of a topical supersaturated pharmaceutical composition comprising:

a) producing a pharmaceutical composition comprising:

i) a pharmaceutically active agent, and

ii) a pharmaceutically acceptable vehicle,

the pharmaceutical composition having a pH of 7 to 12 or a pH of 2 to 4,
characterised in that the pharmaceutically active agent is dissolved at or below its saturation concentration and that the composition becomes supersaturated when the pH is changed to below 7 or greater than 4; and

b) applying said pharmaceutical composition to the skin such that the pH of the pharmaceutical composition will be changed to below 7 or above 4.

Yet further according to the invention there is provided a process for the systemic or local application of an active agent to a human or animal body comprising applying to an area of the surface of the body a topical pharmaceutical composition comprising:-

i) a pharmacologically effective amount of a pharmaceutically active agent, and

ii) a pharmaceutically acceptable vehicle,

the composition having a pH of 7 to 12 or a pH of 2 to 4,
characterised in that the pharmaceutically active agent is dissolved at or below its saturation concentration, such that the composition becomes supersaturated when the pH changes to below 7 or above 4 consequent upon its application to the said surface of a body.

Preferably the topical pharmaceutical compositions of the invention have a pH of from 8 to 10 or from 2.5 to 3.5.

Preferably the topical pharmaceutical compositions of the invention further comprise an anti-nucleating agent, enabling substantial reduction in drug precipitation when the composition becomes supersaturated. In addition, the incorporation of an anti-nucleating agent, by stabilising the supersaturated state, enables still higher degrees of supersaturation of the active agent.

The anti-nucleating agent should be soluble in the composition. Examples of suitable anti-nucleating agents include pharmaceutically acceptable hydroxyalkylcelluloses, such as hydroxypropylmethyl cellulose and hydroxypropyl cellulose; polyvinyl pyrrolidone; polyacrylic acids; condensed phosphates; organic phosphonates; polysulphonates and polymaleates. A mixture of two or more different anti-nucleating agents may be used.

Preferably, when present the anti-nucleating agent is used in an amount of up to 20%, more preferably from 0.01 to 5.0%, most preferably from 0.1 to 2.0% by weight, based on the total weight of the composition.

The choice of suitable anti-nucleating agent will depend upon the particular active agent and the particular vehicle being used, but suitable anti-nucleating agents can readily be determined by simple experiment. This may be done, for example, by preparing samples of the desired final supersaturated active agent solution containing a selection of anti-nucleating agents (in say 1% concentration), one to each sample; allowing the samples to stand for approximately two hours and noting which solutions have remained clear, and thus stable. Further standard techniques may be used to quantify the effect observed.

For a better understanding of the invention reference will be made to the following drawings in which:

Figure 1 is a graph of ketoprofen solubility at different pH values;

Figure 2 is a graph of ketoprofen solubility at different pH values and also the potential degrees of supersaturation achievable at various pH values;

Figure 3 is a graph of the variation of ketoprofen concentration in a 4 fold supersaturated composition over time;

Figure 4 is a graph of triclosan solubility, and the consequent potential degrees of supersaturation achievable, at various pH values;

Figure 5 is a graph showing the buffering capacity of excised human skin; and

Figure 6 is a graph showing the buffering capacity of human skin in vivo.

A very wide range of active agents and vehicles may be used in the topical pharmaceutical compositions of the invention; the only criteria, other than pharmaceutical acceptability, being that the active agent is soluble in the vehicle at a pH within the range of 7 to 12 or 2 to 4 and substantially less soluble in the vehicle at a pH of below 7 or above 4.

The degree of improvement in active agent penetration will depend largely upon the ratio of the supersaturated concentration (i.e. the actual concentration of the active agent in the composition after the pH change on the skin) to the saturation concentration (the theoretical concentration at which the vehicle would be saturated with the active agent at the particular pH of the composition on the skin).

Improvements in penetration may be produced at any ratios greater than 1:1. For slow penetration ratios of from 2:1 to 10:1 may be useful and for rapid penetration ratios of greater than 10:1 may be useful.

The topical pharmaceutical compositions of the present invention may produce extremely high ratios of supersaturation of from 2:1 to 500:1, preferably from 2:1 to 50:1.

It will be clear to those skilled in the art that the selection of active agent, active agent concentration and vehicle will be inter-related, each depending to some extent upon the properties of the other. Normally a suitable active agent will be selected and the concentration thereof, plus the specific vehicle or mixtures thereof, will be chosen such that the active agent will exhibit the solubility and supersaturation properties required in the compositions of the invention. Such combinations may easily be selected by simple experimentation or by the use of published values for solubility etc.

Suitable vehicles for use in the topical pharmaceutical compositions of the invention will be those in which the active agents will be ionisable. More preferably the vehicles will be at least partly aqueous and most preferably they will be predominantly water.

It will be understood that the vehicles used in the topical pharmaceutical compositions of the invention may be mixtures of two or more components as long as all of these components are miscible. Whilst it is most preferred that the vehicles of the compositions of the invention are water they may also be for example mixtures of water and alcohols ( e.g. ethanol or propylene glycol).

Preferably the topical pharmaceutical compositions of the invention are substantially free of volatile solvents.

Preferred active agents for use in the topical pharmaceutical compositions of the invention are those which are ionised in the pH range of the compositions (i.e. 2 to 4 or 7 to 12), more preferably they should be ionised at pH's between 2.5 to 3.5 or 8 to 10. Furthermore, it is also preferred that the active agents will be substantially unionised at the pH range of the compositions after application to the surface of a body (i.e. below 7 or above 4).

More preferably the active agents used in the topical pharmaceutical compositions of the invention will be acids with a pKa of 6.5 to 10 (most preferably 7.5 to 9.5), bases with a pKa of 3 to 4.5 (most preferably 3 to 4), or amphoteric agents with an acid pKa of 6.5 to 10 and/or a base pKa of 3 to 4.5 (most preferably 7.5 to 9.5 and/or 3 to 4).

Most preferably the active agents used in the topical pharmaceutical compositions of the invention will be substantially ionised at some point between the pH range 7.5 to 10 and be substantially unionised over the pH range 4.5 to 6.5. In this case the initial pH of the compositions of the invention will be between 8 and 10.

The concentration of active agent in the topical pharmaceutical compositions of the invention will be selected such that the change in solubility following the pH change after application of the composition to a body surface will be sufficient to produce a supersaturated solution capable of delivering an effective dose of the active agent to the said body surface.

Active agent concentrations of anything from 0.00001 to 50% w/w or higher may therefore be suitable, depending upon the potency of the active agent and its relative solubilities at the initial pH of the composition and at the pH of the body surface. Furthermore, different active agents will be suitable for different delivery means.

For local application, the following classes of active agents are preferred in the topical pharmaceutical compositions of the invention: antimicrobial agents (e.g. antibacterial, antifungal or antiviral agents), steroids, antipsoriasis agents, antiacne agents, local anaesthetics, non steroidal anti inflammatory agents, antidandruff agents, headlice treating agents and antihistamines.

Preferred antimicrobial agents include triclosan (preferably 0.01 to 2.5%w/w), hexylresorcinol (preferably 0.05 to 5%w/w), miconazole (preferably 0.1 to 4%w/w), acyclovir (preferably 0.1 to 5% w/w), metronidazole (preferably 0.01 to 8%w/w), 4-chloro-3-methylphenol (preferably 0.1 to 10% w/w), 4-chloro-3,5-dimethylphenol (preferably 0.1 to 10% w/w), chlorocresol (preferably 0.001 to 2% w/w) and 2,4-dichloro-3,5-dimethylphenol (preferably 0.1 to 10%w/w).

A preferred steroid is hydrocortisone (preferably 0.1 to 5%w/w).

Preferred antipsoriasis agents include methotrexate (preferably 0.001 to 0.5%w/w), dithranol (preferably 0.001 to 5% w/w) and calcipotriol (preferably 0.00005 to 0.05% w/w).

Preferred antiacne agents are retinoic acid and analogues thereof (preferably 0.00001 to 5%w/w).

A preferred local anaesthetic is benzocaine (preferably 0.1 to 5%w/w).

Preferred non steroidal anti-inflammatory agents include ketoprofen (preferably 0.5 to 5%w/w), piroxicam (preferably 0.01 to 2%w/w) and diclofenac (preferably 0.1 to 5%w/w).

A preferred antidandruff agent is zinc omadine (preferably 0.01 to 10%w/w).

A preferred headlice treating agent is an acaricide (preferably 0.05 to 5% w/w).

Preferred antihistamines include mepyramine (preferably 0.1 to 5%w/w), cimetidine (preferably 0.01 to 10% w/w) and terfenadine (preferably 0.1 to 4% w/w).

For systemic application, the following classes of active agents are preferred in the topical pharmaceutical compositions of the invention: anti travel sickness agents, bronchospasm relaxants, antihistamines, decongestants, antitussives, analgesics, anticoagulants, beta adrenoceptor blockers, anti angina agents, anti emetics, antimicrobial agents, bronchodilators, anti allergy agents, anti migraine agents, corticosteroids and thyroid agents.

Preferred analgesics include indomethacin (preferably 0.01 to 1%w/w) and naproxen (preferably 0.1 to 2%w/w)

Preferred anticoagulants include warferin (preferably 0.001 to 0.1%w/w).

Preferred antimicrobial agents include triclosan (preferably 0.001 to 1.0 w/w).

Preferred bronchodilators include beclomethasone (preferably 0.001 to 0.05% w/w), ipratropium (preferably 0.0001 to 0.01% w/w).

Preferred antiallergy agents include ketotifen (preferably 0.001 - 0.1% w/w).

Preferred antimigraine agents include ergotamine (preferably 0.0005 to 0.5% w/w).

Preferred corticosteroids include dexamethasone (preferably 0.0005 - 0.5% w/w) and prednisolone (preferably 0.001 - 0.5% w/w).

Other preferred systemic active agents for use in the topical pharmaceutical compositions of the invention include acetylcysteine, an antidote to paracetamol poisoning (at 2 to 100% w/w); diazepam, an anxiolytic agent (at 0.01 to 10% w/w); nicotinamide, a vitamin (at 2.5 to 50% w/w); and phenytoin, an antiepilepsy agent (at 2.5 to 50% w/w).

More preferred agent for use in the topical pharmaceutical compositions of the invention include triclosan, hexylresorcinol, miconazole, acyclovir, metronidazole, 4-chloro-3-methyl phenol, 4-chloro-3,5-dimethyl phenol, 2,4-dichloro-3,5-dimethyl phenol, hydrocortisone, methotrexate, dithranol, calcipotriol, retinoic acid, benzocaine, ketoprofen, piroxicam, diclofenac, mepyramine, terfenadine, cimetidine, indomethacin, naproxen, warferin, ketotifen, ergotamine, acetylcysteine, chlorocresol, diazepam, nicotinamide and phenytoin; most preferably triclosan, hexylresorcinol, dithranol, retinoic acid, acetylcysteine, chlorocresol, diazepam, nicotinamide and phenytoin.

The topical pharmaceutical compositions of the invention may suitably be applied to any part of the body having sufficient buffering capacity to effect a suitable pH change. This includes all regions of the skin and the mucous membranes (e.g. the vagina, nasal passage or mouth).

Preferably the topical pharmaceutical compositions according to the invention may further include one or more of the following agents selected from:-

    i) a thickener (preferably a carbomer, e.g. Carbopol 940) - preferably in an amount of 0.1 to 5.0% w/w),

    ii) a humectant, preferably selected from glycerol, sorbitol, propylene glycol and tricetin, (more preferably glycerol), preferably in an amount of 0.1 to 20% w/w; and

    iii) a solubiliser to enhance the solubility of the active agent before application to the surface of a body (preferably propylene glycol) - preferably in an amount of 0.1 to 50% w/w).

Optionally, the topical pharmaceutical compositions may contain a penetration enhancer, e.g. Azone, or terpenes at a preferred amount of 0.1 to 10% w/w; or diethylene glycol monoethyl ether at a concentration of 5 to 50%.

Preferably the topical pharmaceutical compositions according to the invention contain 30 - 99.5% w/w water.

Preferably the topical pharmaceutical compositions of the invention are free from agents which might act to counter the pH change upon application to the skin (i.e. buffering agents) other than any inherent buffering capacity bestowed by the active agent.

If necessary the topical pharmaceutical compositions of the invention may comprise pH adjusting agents in order that the pH before application to the skin is in the range of 2 to 4 or 7 to 12.

The topical pharmaceutical compositions of the invention may be in any conventional form suitable for topical application, i.e. liquids, gels, suspensions, ointments or collodions. They may also be applied to the surface of a body as a transdermal patch or predispersed on a carrier, for example as a medicated plaster.

The topical pharmaceutical compositions of the invention may be manufactured by any suitable conventional means. For example the active agent may be dissolved in the vehicle (optionally with the aid of one or more solubilisers); any other optional soluble components may be added; the pH adjusted if necessary and any non soluble or thickening agents added.

As the topical pharmaceutical compositions of the invention are single phase, generally non volatile, compositions they do not require sophisticated packaging. There is therefore yet further provided a pharmaceutical package comprising a topical pharmaceutical composition of the invention which package does not contain any other components/phases.

The invention will be illustrated by the following Examples:

Example 1

Effect of pH on ketoprofen solubility

Run 1

A range of solutions comprising mixtures of 0.1M citric acid and 0.2M $Na_2HPO_4$, were produced having the pH's shown in Table 1. A weight of ketoprofen, as shown in Table 1, was add to a 20 ml sample of each solution and the mixture was stirred for 24 hours at 25°C. The pH of the final solution was measured.

Table 1

| Original pH | Amount of Ketoprofen added (g) | pH on sampling after 24 hours |
|---|---|---|
| 3 | 0.1 | 3 |
| 4 | 0.1 | 4 |
| 5 | 0.1 | 4.9 |
| 5.5 | 0.5 | 5.4 |
| 6 | 0.5 | 5.8 |
| 6.5 | 0.5 | 6 |
| 7 | 0.5 | 6.2 |
| 7.5 | 0.5 | 6.3 |

The concentration of ketoprofen dissolved at each pH was measured by UV spectrophotometry (at 258nm) following filtration through a 0.2 um syringe filter to remove undissolved material.

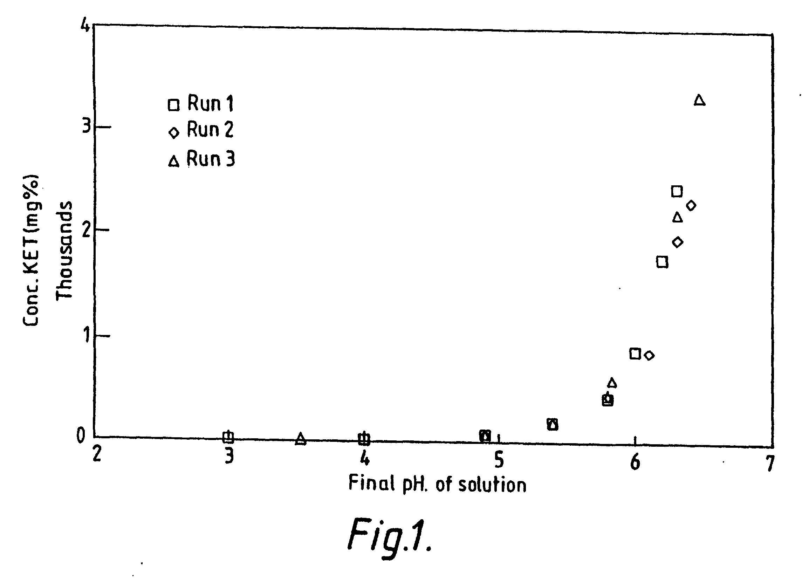

The results are shown in Figure 1.

Run 2

Run 1 was repeated with the exception of the solutions at pH 3 and 4, as shown in Table 2.

Table 2

| Original pH | Amount of Ketoprofen added (g) | pH on sampling after 24 hours |
|---|---|---|
| 5 | 0.1 | 4.9 |
| 5.5 | 0.5 | 5.4 |
| 6 | 0.5 | 5.8 |
| 6.5 | 0.5 | 6.1 |
| 7 | 0.5 | 6.3 |
| 7.5 | 0.5 | 6.4 |

The results are shown in figure 1.

Run 3

Saturated solutions of ketoprofen were prepared by a different method to runs 1 and 2 to act as a check on methodology.

4g of ketoprofen was added to 100 ml of deionised water and stirred at 25°C for 24 hours. The pH was tested and found to be 3.5, a sample was taken.

The mixture was then adjusted to a pH of 5.8 with 1M NaOH and stirred for a further 24 hours at 25°C, after which a sample was removed.

The mixture was adjusted to a pH of 6.4 using 1M NaOH and again stirred for 24 hours at a temperature of 25°C. A final sample was removed.

The three samples were filtered to remove undissolved ketoprofen (0.2 um filter) and the concentration of dissolved ketoprofen measured by the same method as in Runs 1 and 2.

The results are shown in figure 1.

Figure 1 demonstrates the vast change in ketoprofen solubility caused by pH changes. Similar results may be produced for a range of ionisable active agents, and may be calculated from a solubility equation, with a knowledge of the active agents' pH and solubility in the unionised form (Physicochemical Principles of Pharmacy 2nd Edition, Florence and Attwood, 1988).

Example 2

Demonstration of Theoretical Supersaturation Achievable from Alteration of Solution pH.

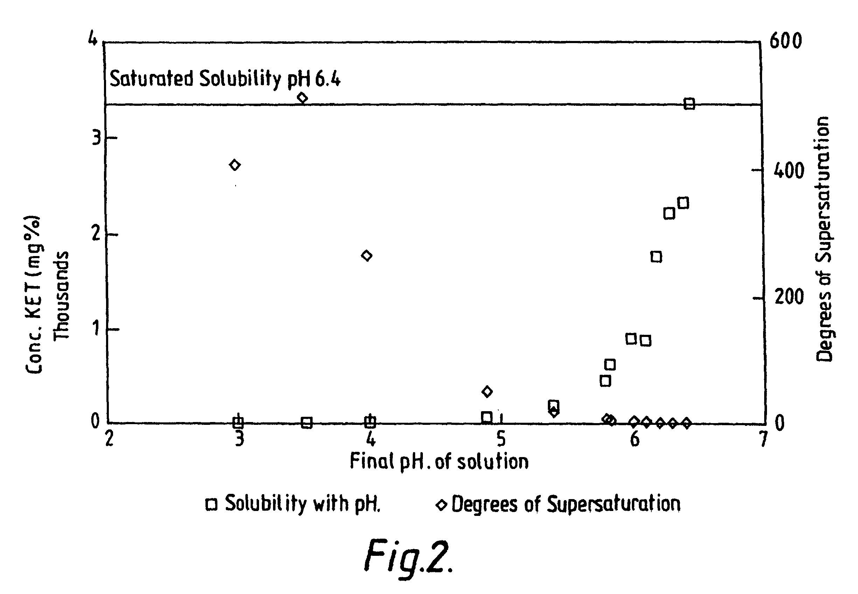

Figure 2 demonstrates the potential degrees of supersaturation achievable, with ketoprofen as the example active agent, as the pH is reduced from pH 6.4 (example starting point pH).

The potential degree of supersaturation achievable at each pH is determined by dividing the saturated solubility of ketoprofen at pH 6.4 with the saturated solubility at each pH value of interest, where the solubility is lower than that observed at pH 6.4.

In the example shown in Figure 2, the maximum theoretical degree of supersaturation achievable with ketoprofen is 513X. The maximum theoretical degree of supersaturation achievable at skin pH (for example pH 5.5) is 18X. it is to be understood that further increases in theoretical degrees of supersaturation could be achieved by increasing the starting point pH until still higher ketoprofen solubility is achieved.

The ketoprofen solubility values for pH 4.9, 5.4, 5.8 and 6.3 shown on figure 2 are averages of the equivalent concentrations from runs 1 and 2. All other values are the same as in Example 1.

Example 3

Stabilisation of a supersaturated solution.

An aqueous solution of 0.8% w/v ketoprofen at a pH of 10.4 was prepared at 25°C. 1% polyvinyl pyrrolidone (PVP) was added to the solution. Aliquots of 0.1M hydrochloric acid were added to the solution with stirring until a pH of 5.5 was achieved. A 4X supersaturated solution of ketoprofen was thus produced.

The amount of ketoprofen dissolved in the solution was measured at various times by the method used in Example 1 (after filtration through a 0.2um filter to remove undissolved ketoprofen).

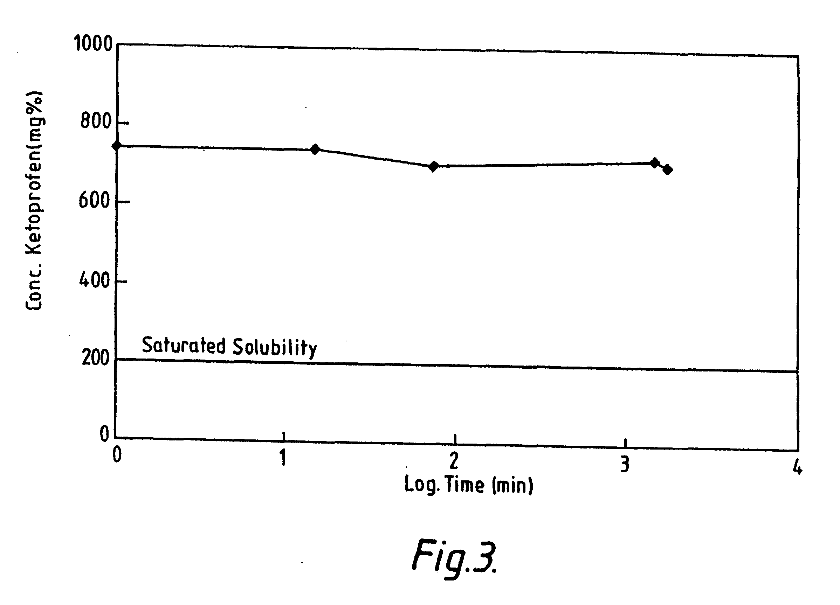

The results are shown in Figure 3, which shows that supersaturation was maintained for at least 16 hours.

Example 4

A range of solutions having the compositions and pHs as shown in Table 3 were prepared. The amounts of triclosan as shown in Table 3 were added to 20 ml of each solution and the mixtures were stirred for 48 hours at 37°C. the final pH of each solution was measured.

Table 3

| Original pH | Buffer Composition | Amount of Triclosan Added g | pH on Sampling after 48 hrs |
|---|---|---|---|
| 4 | 0.1M citric acid 0.2M $Na_2HPO_4$ | 0.1 | 4.1 |
| 5 | 0.1M citric acid 0.2M $Na_2HPO_4$ | 0.1 | 5.2 |
| 6 | 0.1M citric acid 0.2M $Na_2HPO_4$ | 0.5 | 6.3 |
| 7 | 0.2M $NaH_2PO_4$ | 0.5 | 7.1 |

Table 3   (continued)

| Original pH | Buffer Composition | Amount of Triclosan Added g | pH on Sampling after 48 hrs |
|---|---|---|---|
| 8 | 0.2M $NaH_2PO_4$ 0.2M $Na_2HPO_4$ | 0.5 | 8.1 |
| 9.2 | 0.2M $NaH_2PO_4$ 0.1M $Na_2CO_3$ | 1 | 9.6 |
| 10.1 | 0.1M $NaHCO_3$ 0.1M $Na_2CO_3$ 0.1M $NaHCO_3$ | 1 | 10.6 |

Samples of each mixture were centrifuged at 3000 rpm for 10 minutes to remove undissolved material. The concentration of dissolved triclosan was measured in each solution by HPLC using a standard procedure (detection at 281nm).

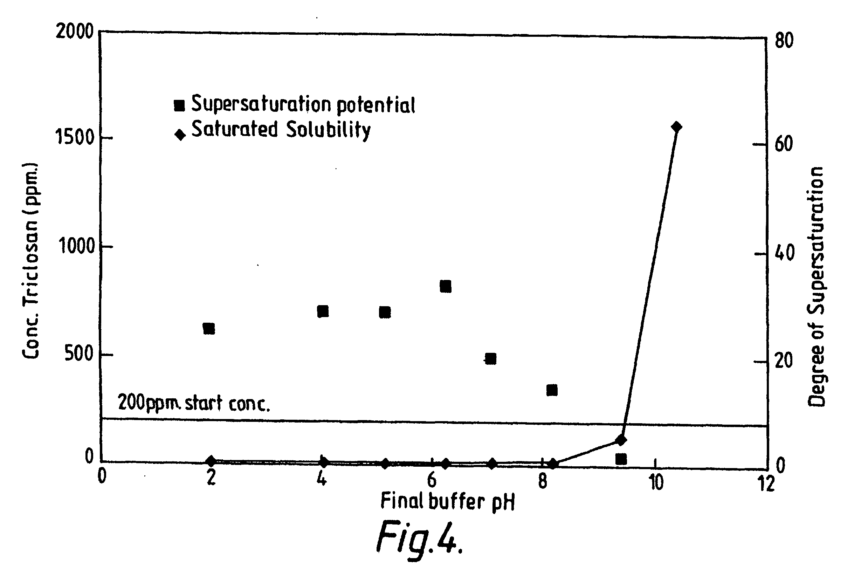

The solubility of triclosan at each pH is shown in Figure 4.

The supersaturation potential of a solution at pH 10 containing 200ppm triclosan was calculated by dividing the concentration of triclosan in that solution by the concentration of triclosan in the solutions at lower pHs.

These theoretical values are also shown in Figure 4. Thus it can be seen that the degree of supersaturation achievable at a skin pH (e.g. pH 5.5) is 30X if a 200ppm triclosan solution at pH 10 is used as the starting composition. It is to be understood that higher degrees of supersaturation may be achieved by increasing the triclosan concentration in solution at pH 10, or further by increasing the pH of the starting composition.

Example 5

| Topical gel | % w/w |
|---|---|
| Acyclovir sodium salt | 2.0 |
| Carbopol 940 | 1.0 |
| Polyvinyl pyrrolidone | 1.0 |
| Propylene glycol | 10.0 |
| Deionised water | 86.0 |
| | 100.00 |

The acyclovir sodium is dispersed in the propylene glycol. In a separate vessel the carbopol and the polyvinyl pyrrolidone are dispersed in the water. The two phases are combined and the pH is adjusted to ensure a final pH of 10.0.

Upon application to the skin the change in pH of the composition to 6.5 or below will result in the formation of an approximately 10X supersaturated composition.

Example 6

| Topical gel | % w/w |
|---|---|
| Piroxicam | 0.2 |
| Carbopol 940 | 1.0 |
| Hydroxypropylmethyl cellulose | 2.0 |
| Triethanolamine | 0.2 |
| Propylene glycol | 50.0 |
| Deionised water | 46.6 |
| | 100.00 |

The piroxicam, carbopol 940 and the hydroxypropylmethyl cellulose are dispersed in the propylene glycol.

The water and triethanolamine are added and mixed well and the pH is adjusted to ensure a final pH of 9.

Upon application to the skin, the change in pH of the composition to 6.5 or below will result in the formation of an approximately 10X supersaturated composition.

Example 7

The ability of excised, frozen and thawed human skin to alter the pH of a simple formulation placed in contact with the skin surface.

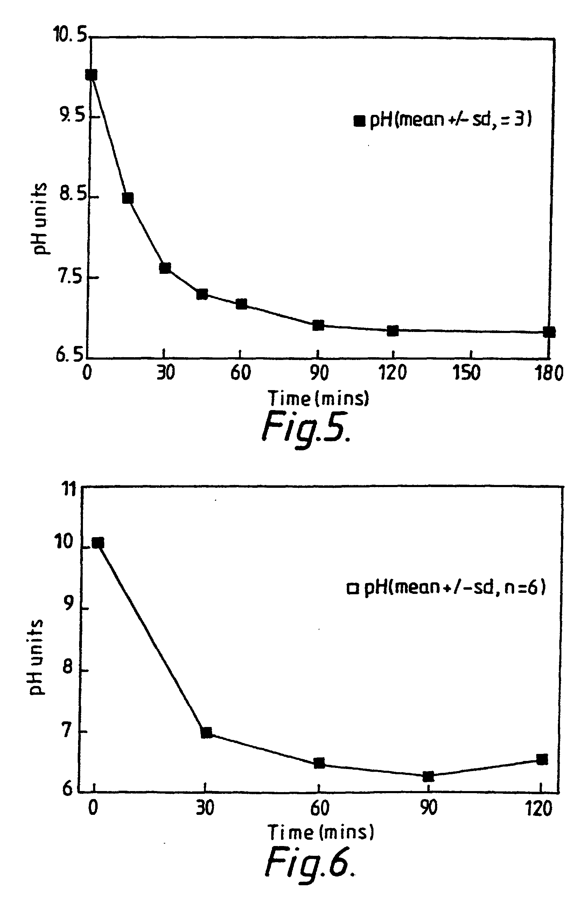

Female human breast skin was thoroughly defrosted at room temperature and the surface was swabbed with distilled water and then gently dried. A solution of sodium hydroxide in distilled water was prepared so that its pH was 10. Specially constructed translucent polypropylene cells were greased with silicone grease to prevent leakages and were then placed onto the skin surface so as to form a sealed cell, the base of which was the skin. The cells were clamped in place using aluminium spring clips. At the beginning of the experiment 5 mls of the pH 10 sodium hydroxide solution was introduced into the cell so that the solution was in contact with 2.98 cm$^2$ of the skin surface. The upper opening of the cell was then sealed from the atmosphere with nescofilm (Registered Trade Mark). The pH of the solution held in the cell was periodically measured using a small Whatman pH electrode attached to a Whatman pH meter and recorded. The results are shown in Figure 5.

Figure 5 demonstrates that within 2 hours of contact between a simple formulation and excised human skin, the pH of the formulation fell by more than 3 pH units from pH 10.01 to pH 6.84.

Example 8

The ability of human skin, in vivo to alter the pH of a simple formulation placed in contact with the skin surface.

The volar aspect of the left forearm of two volunteers was swabbed with distilled water and patted dry. A solution of sodium hydroxide in distilled water was prepared so that its pH was 10. Three specially constructed translucent polypropylene cells were greased with silicone grease to prevent leakages and were then placed onto the skin surface so as to form a sealed cell, the base of which was the skin. The cells were held in place using tubigrip (Registered Trade Mark) dressings with a suitable sized aperture cut into it. At the beginning of the experiment 5 mls of the pH 10 sodium hydroxide solution was introduced into the cell so that the solution was in contact with 2.98 cm$^2$ of the skin surface. The upper opening of the cell was then flushed with nitrogen gas and sealed from the atmosphere with nescofilm (Registered Trade Mark). The pH of the solution held in the cell was periodically measured using a small Whatman pH electrode attached to a Whatman pH meter and recorded. After each pH measurement the cell was flushed with nitrogen gas and sealed from the atmosphere with nescofilm (Registered Trade Mark). The results are shown in Figure 6.

Figure 6 demonstrates that within 90 minutes of contact between a simple formulation and living human skin in situ, the pH of the formulation fell by more than 3.5 pH units from pH 10.08 to pH 6.26.

Example 9

| Topical Gel | |
|---|---|
| | % w/w |
| Triclosan | 0.2 |
| Carbopol 940 | 1.0 |
| Hydroxypropylmethyl Cellulose | 1.0 |
| Deionised Water | 97.8 |
| | $\overline{100}$ |

The triclosan, carbopol and hydroxypropylmethyl cellulose are dispersed in the water and the pH is adjusted to give a gel of pH 10.

Upon application to the skin, the pH of the composition is reduced to below 7 resulting in the formation of an approximately 10X supersaturated composition.

Example 10

| Topical Gel | |
| --- | --- |
| | % w/w |
| Benzocaine | 0.3 |
| Carbopol 940 | 1.0 |
| Polyvinyl pyrrolidone | 1.0 |
| Propylene glycol | 10.0 |
| Deionised water | 87.7 |
| | 100 |

The benzocaine is dispersed in the propylene glycol. In a separate vessel the carbopol and polyvinyl pyrrolidone are dispersed in the water. The two phases are mixed and the pH is adjusted to a final pH of 2.

**Claims**

1. A pharmaceutical composition for topical application comprising

    i) a pharmaceutically active agent, and

    ii) a pharmaceutically acceptable vehicle,

    the pharmaceutical composition having a pH of 7 to 12 or a pH of 2 to 4,
    **characterised in that** the pharmaceutically active agent is dissolved at or below its saturation concentration and that the composition becomes supersaturated when the pH is changed to below 7 or greater than 4.

2. A topical pharmaceutical composition as claimed in Claim 1 wherein the pH is 8 to 10 or 2.5 to 3.5.

3. A topical pharmaceutical composition as claimed in Claim 1 or Claim 2 further comprising an anti-nucleating agent.

4. A topical pharmaceutical composition as claimed in any previous Claim wherein the vehicle is at least partly aqueous.

5. A topical pharmaceutical composition as claimed in any previous Claim wherein the pharmaceutically active agent is selected from acids with a pKa of 6.5 to 10, bases with a pKa of 3 to 4.5, or amphoteric agents with an acid pKa of 6.5 to 10 and/or a base pKa of 3 to 4.5.

6. A topical pharmaceutical composition as claimed in any previous Claim wherein the concentration of the pharmaceutically active agent is from 0.00001 to 50% w/w.

7. A topical pharmaceutical composition as claimed in any previous Claim further comprising one or more of the following agents:-

    i) a thickener,

    ii) a humectant, and

    iii) a solubiliser to enhance the solubility of the active agent before application to the surface of a body.

8. A topical pharmaceutical composition as claimed in any previous Claim further comprising a penetration enhancer.

9. A topical pharmaceutical composition as claimed in any previous Claim comprising 30 - 99.5% w/w water.

10. A topical pharmaceutical composition as claimed in any previous Claim in the form of a liquid, a gel, a suspension, an ointment, a collodion; or in the form of a medicated plaster or a transdermal patch.

11. A process for the preparation of a topical supersaturated pharmaceutical composition comprising:

   a) producing a pharmaceutical composition comprising:

   i) a pharmaceutically active agent, and

   ii) a pharmaceutically acceptable vehicle,

   the pharmaceutical composition having a pH of 7 to 12 or a pH of 2 to 4,
   **characterised in that** the pharmaceutically active agent is dissolved at or below its saturation concentration and that the composition becomes supersaturated when the pH is changed to below 7 or greater than 4; and

   b) applying said pharmaceutical composition to the skin such that the pH of the pharmaceutical composition will be changed to below 7 or above 4.

12. A process for the preparation of a topical pharmaceutical composition comprising:-

   i) a pharmacologically effective amount of a pharmaceutically active agent, and

   ii) a pharmaceutically acceptable vehicle,

   the composition having a pH of 7 to 12 or a pH of 2 to 4,
   **characterised in that** the pharmaceutically active agent is dissolved at or below its saturation concentration, such that the composition becomes supersaturated when the pH changes to below 7 or above 4 consequent upon its application to the said surface of a body for the systemic or local application of an active agent to a human or animal body by application to an area of the surface of the body.

13. A process as claimed in Claims 11 or 12 wherein the topical pharmaceutical composition is applied to any part of the body having sufficient buffering capacity to effect a suitable pH change.

14. A pharmaceutical package comprising a topical pharmaceutical composition as claimed in any of Claims 1 to 10 which package does not contain any other components/phases.


**Patentansprüche**

1. Arzneimittel zur topischen Anwendung umfassend:

   i) einen pharmazeutischen Wirkstoff, und
   ii) einen pharmazeutisch verträglichen Träger,

   wobei das Arzneimittel einen pH-Wert von 7 bis 12 oder einen pH-Wert von 2 bis 4 aufweist,
   **dadurch gekennzeichnet, dass** der pharmazeutische Wirkstoff bei oder unterhalb seiner Sättigungskonzentration gelöst ist, und dass die Zusammensetzung übersättigt wird, wenn der pH-Wert auf unter 7 oder größer als 4 geändert wird.

2. Topisches Arzneimittel nach Anspruch 1, wobei der pH-Wert 8 bis 10 oder 2,5 bis 3,5 ist.

3. Topisches Arzneimittel nach Anspruch 1 oder 2, das weiterhin ein Anti-Keimbildungsmittel umfasst.

4. Topisches Arzneimittel nach einem der vorhergehenden Ansprüche, wobei der Träger mindestens teilweise wässrig ist.

5. Topisches Arzneimittel nach einem der vorhergehenden Ansprüche, wobei der pharmazeutische Wirkstoff ausgewählt ist aus Säuren mit einem pKa-Wert von 6,5 bis 10, Basen mit einem pKa-Wert von 3 bis 4,5 oder amphoteren Mitteln mit einem Säure-pKa-Wert von 6,5 bis 10 und/oder einem Basen-pKa-Wert von 3 bis 4,5.

6. Topisches Arzneimittel nach einem der vorhergehenden Ansprüche, wobei die Konzentration des pharmazeutischen Wirkstoffs 0.00001 bis 50% Gew./Gew. ist.

7. Topisches Arzneimittel nach einem der vorhergehenden Ansprüche, das weiterhin eines oder mehrere der folgenden Mittel umfasst:

   i) ein Verdickungsmittel,
   ii) ein Feuchthaltemittel, und
   iii) ein Auflösungsmittel, um die Löslichkeit des Wirkstoffs vor dem Aufbringen auf die Oberfläche eines Körpers zu erhöhen.

8. Topisches Arzneimittel nach einem der vorhergehenden Ansprüche, das weiterhin einen Eindringverstärker umfasst.

9. Topisches Arzneimittel nach einem der vorhergehenden Ansprüche, das 30 bis 99, 5% Gew./Gew. Wasser umfasst.

10. Topisches Arzneimittel nach einem der vorhergehenden Ansprüche in Form einer Flüssigkeit, eines Gels, einer Suspension, einer Salbe, eines Kollodiums, oder in Form eines wirkstoffhaltigen Pflasters oder eines transdermalen Pflasters.

11. Verfahren zur Herstellung eines übersättigten topischen Arzneimittels umfassend:

   a) Herstellen eines Arzneimittels umfassend:

      i) einen pharmazeutischen Wirkstoff, und
      ii) einen pharmazeutisch verträglichen Träger,

   wobei das Arzneimittel einen pH-Wert von 7 bis 12 oder einen pH-Wert von 2 bis 4 aufweist, **dadurch gekennzeichnet, dass** der pharmazeutische Wirkstoff bei oder unterhalb seiner Sättigungskonzentration gelöst ist, und dass die Zusammensetzung übersättigt wird, wenn der pH-Wert auf unter 7 oder größer als 4 geändert wird; und
   b) Aufbringen des Arzneimittels auf die Haut, so dass der pH-Wert des Arzneimittels auf unter 7 oder über 4 geändert wird.

12. Verfahren zur Herstellung eines topischen Arzneimittels umfassend:

   i) eine pharmakologisch wirksame Menge eines pharmazeutischen Wirkstoffs, und
   ii) einen pharmazeutisch verträglichen Träger,

   wobei das Arzneimittel einen pH-Wert von 7 bis 12 oder einen pH-Wert von 2 bis 4 aufweist, **dadurch gekennzeichnet, dass** der pharmazeutische Wirkstoff bei oder unterhalb seiner Sättigungskonzentration gelöst wird, so dass die Zusammensetzung übersättigt wird, wenn sich der pH-Wert nach ihrem Aufbringen auf die Oberfläche eines Körpers zur systemischen oder lokalen Anwendung eines Wirkstoffs auf einen menschlichen oder tierischen Körper beim Aufbringen auf einem Bereich einer Oberfläche des Körpers auf unter 7 oder über 4 ändert.

13. Verfahren nach Anspruch 11 oder 12, wobei das topische Arzneimittel auf einem beliebigen Teil des Körpers aufgebracht wird, der ausreichende Pufferkapazität aufweist, um eine geeignete pH-Wert-Änderung zu bewirken.

14. Pharmazeutische Packung umfassend ein topisches Arzneimittel nach einem der Ansprüche 1 bis 10, wobei die Packung keine anderen Bestandteile/Phasen enthält.

**Revendications**

1. Composition pharmaceutique pour application topique, comprenant

i) un agent pharmaceutiquement actif, et

ii) un véhicule pharmaceutiquement acceptable

la composition pharmaceutique ayant un pH de 7 à 12 ou un pH de 2 à 4,

**caractérisée en ce que** l'agent pharmaceutiquement actif est dissous à une concentration inférieure ou égale à sa concentration à saturation et **en ce que** la composition devient sursaturée lorsque le pH est amené à une valeur inférieure à 7 ou supérieure à 4.

**2.** Composition pharmaceutique topique suivant la revendication 1, dans laquelle le pH est compris dans la plage de 8 à 10 ou de 2,5 à 3,5.

**3.** Composition pharmaceutique topique suivant la revendication 1 ou la revendication 2, comprenant en outre un agent antinucléation.

**4.** Composition pharmaceutique topique suivant l'une quelconque des revendications précédentes, dans laquelle le véhicule est au moins partiellement aqueux.

**5.** Composition pharmaceutique topique suivant l'une quelconque des revendications précédentes, dans laquelle l'agent pharmaceutiquement actif est choisi parmi des acides ayant un pKa de 6,5 à 10, des bases ayant un pKa de 3 à 4,5 ou des agents amphotères ayant un pKa acide de 6,5 à 10 et/ou un pKa basique de 3 à 4,5.

**6.** Composition pharmaceutique topique suivant l'une quelconque des revendications précédentes, dans laquelle la concentration d'agent pharmaceutiquement actif est comprise dans l'intervalle de 0,00001 à 50 % en poids/poids.

**7.** Composition pharmaceutique topique suivant l'une quelconque des revendications précédentes, comprenant en outre un ou plusieurs des agents suivants :

i) un épaississant,

ii) un agent humectant, et

iii) un solubilisant pour accroître la solubilité de l'agent actif avant l'application à la surface d'un corps.

**8.** Composition pharmaceutique topique suivant l'une quelconque des revendications précédentes, comprenant en outre un activateur de pénétration.

**9.** Composition pharmaceutique topique suivant l'une quelconque des revendications précédentes, comprenant 30 à 99,5 % en poids/poids d'eau.

**10.** Composition pharmaceutique topique suivant l'une quelconque des revendications précédentes, sous forme d'un liquide, d'un gel, d'une suspension, d'une pommade, d'un collodion ; ou sous forme d'un emplâtre médicamenteux ou d'un timbre transcutané.

**11.** Procédé pour la préparation d'une composition pharmaceutique sursaturée topique, comprenant les étapes consistant :

a) à produire une composition pharmaceutique comprenant :

i) un agent pharmaceutiquement actif, et

ii) un véhicule pharmaceutiquement acceptable

la composition pharmaceutique ayant un pH de 7 à 12 ou un pH de 2 à 4,

**caractérisé en ce que** l'agent pharmaceutiquement actif est dissous à une concentration inférieure ou égale à sa concentration à saturation et **en ce que** la composition devient sursaturée lorsque le pH est porté à une valeur inférieure à 7 ou une valeur supérieure à 4 ; et

b) à appliquer ladite composition pharmaceutique à la peau de telle sorte que le pH de la composition pharmaceutique soit porté à une valeur inférieure à 7 ou supérieure à 4.

**12.** Procédé pour la préparation d'une composition pharmaceutique topique, comprenant :

i) une quantité pharmacologiquement efficace d'un agent pharmaceutiquement actif, et

ii) un véhicule pharmaceutiquement acceptable,

la composition ayant un pH de 7 à 12 ou un pH de 2 à 4,

**caractérisé en ce que** l'agent pharmaceutiquement actif est dissous à une concentration inférieure ou égale à sa concentration à saturation, de telle sorte que la composition devienne sursaturée lorsque le pH est porté à une valeur inférieure à 7 ou supérieure à 4 en conséquence de son application à ladite surface d'un corps pour l'application générale ou locale d'un agent actif au corps de l'homme ou d'un animal par application à une zone de la surface du corps.

13. Procédé suivant la revendication 11 ou 12, dans lequel la composition pharmaceutique topique est appliquée à n'importe quelle partie du corps ayant une capacité tampon suffisante pour provoquer une variation de pH convenable.

14. Conditionnement pharmaceutique comprenant une composition pharmaceutique topique suivant l'une quelconque des revendications 1 à 10, conditionnement qui ne contient aucun autre constituant/aucune autre phase.

*Fig.1.*

Fig.2.

EP 0 862 417 B1

Fig.3.

Fig.4.

Fig.5.

Fig.6.